# EUROPÄISCHE PATENTANMELDUNG

(11) **EP 4 789 761 A1**
(43) Veröffentlichungstag der Anmeldung: **12.08.2026**
(21) Anmeldenummer: 26155738.3
(22) Anmeldetag: 02.02.2026
(51) Int. Cl.: B01J 23/80, B01J 23/06, B01J 23/72, C23C 14/58, B23K 26/352, B23K 26/386, B01J 37/34, C07C 29/154, B01J 37/02

(54) **MEHRSCHICHTKATALYSATOR UND VERFAHREN ZUR UMWANDLUNG VON CO2 UNTER NUTZUNG DES MEHRSCHICHTKATALYSATORS**

(30) Priorität: 05.02.2025 DE 102025104257
(71) Anmelder: Helmholtz-Zentrum Berlin für Materialien und Energie GmbH, 14109 Berlin (DE)
(72) Erfinder: Thum, Lukas, 12489 Berlin (DE); Amkreutz, Daniel, 12489 Berlin (DE); Shivam, Shivam, 12489 Berlin (DE); Schlatmann, Rutger, 12489 Berlin (DE)

(57) **Zusammenfassung**

Die Erfindung betrifft einen Mehrschichtkatalysator (1), aufweisend:
- ein Substrat (2) sowie
- einen auf dem Substrat (2) angeordneten Stapel (4) mit mindestens 10 übereinander angeordneten Schichten , wobei benachbarte Schichten unterschiedliche Materialien wie Cu und ZnO aufweisen, und wobei der Stapel (4) eine Vielzahl von Aussparungen (5) aufweist, oder
auf dem Substrat (2) eine Vielzahl von Stapeln (4', 4", 4‴, 4ʺʺ) angeordnet ist und
wobei der Mehrschichtkatalysator (1) ein Stapeloberfläche-zu-Stapelvolumen-Verhältnis aus einer freiliegenden Oberfläche des Stapels (4) oder der Stapel (4', 4", 4"', 4ʺʺ) zu einem Volumen des Stapels (4) oder der Stapel (4', 4", 4‴, 4ʺʺ) aufweist, das zwischen 100 1/m und 400.000.000 1/m, insbesondere zwischen 10.000 1/m und 4.000.000 1/m beträgt.

Die Erfindung betrifft weiterhin ein Verfahren zur Umwandlung von CO₂ unter Verwendung von Wasserstoff in Gegenwart eines solchen Mehrschichtkatalysators (1).

## Beschreibung

### Technisches Gebiet

Die vorliegende Erfindung bezieht sich auf einen Mehrschichtkatalysator für die heterogene Katalyse, der in mehreren heterogen katalysierten Prozessen eingesetzt werden kann. Zudem bezieht sich die Erfindung auf ein Verfahren zur Umwandlung von CO₂ unter Verwendung von Wasserstoff in Gegenwart eines solchen Mehrschichtkatalysators.

### Hintergrund der Erfindung

Die Entwicklung von Katalysatoren mit maßgeschneiderten Leistungsmerkmalen ist zu einem zentralen Fokus der Katalyseforschung geworden. Einkomponentige Katalysatoren, bzw. geträgerte Metallkatalysatoren, sind oft durch Skalierungsbeziehungen für Adsorptions- und Übergangszustandsenergien begrenzt, was ihre Aktivität und Selektivität auf bestimmte Reaktionen beschränkt. Diese Begrenzung erfordert den Einsatz von Mehrkomponenten-Katalysatoren, die die Eigenschaften chemisch diverser Materialien kombinieren, um die Leistungsgrenzen einkomponentiger Systeme zu übertreffen.

Das Design von Mehrkomponenten-Katalysatoren für die heterogene Katalyse hat großes Interesse geweckt, da sie in der Lage sind, unterschiedliche chemische Eigenschaften synergistisch zu vereinen. Diese Katalysatoren bestehen aus mindestens zwei unterscheidbaren Feststoffkomponenten, die amorphe, kristalline oder gemischte Phasenstrukturen aufweisen können. Die Mehrkomponenten-Natur verbessert die katalytische Aktivität und Selektivität durch kooperative Wechselwirkungen zwischen den Komponenten. Besonders wichtig sind die Kontaktstellen zwischen diesen Komponenten, die eine entscheidende Rolle bei der Bestimmung der Gesamtleistung des Katalysators spielen.

In Mehrkomponentensystemen sind Variationen in der chemischen Zusammensetzung und im strukturellen Zustand häufig und oft beabsichtigt. Unterschiede können durch Variationen in der Stöchiometrie (z. B. Sauerstoffmangel in Oxiden), das Vorhandensein von Dotierstoffen oder strukturelle Zustände wie amorphe oder Flüssigkristallphasen entstehen. Solche Variationen können zwischen verschiedenen Chargen oder sogar innerhalb einer einzigen Katalysatorcharge auftreten und sich während des katalytischen Einsatzes dynamisch entwickeln. Diese Inhomogenität stellt zwar Herausforderungen dar, eröffnet aber auch Möglichkeiten zur Anpassung von Katalysatoren an spezifische Anwendungen.

Beispiele für Mehrkomponenten-Katalysatorsysteme umfassen, sind aber nicht beschränkt auf, Cu/ZnO, CuO/Al₂O₃, Ga-Pd/Ga₂O₃, Co/MnO, Mn-Na-WOx/SiO₂ und Ru/Sm₂O₃.

Besondere Aufmerksamkeit wurde Kombinationen aus Metall und Metalloxid wie Pt/γ-Al₂O₃, Cu/ZnO und Cu/CeO₂/ZSM-5 gewidmet, die aufgrund ihrer synergistischen Wechselwirkungen an den Kontaktstellen bemerkenswerte Leistungen gezeigt haben.

Wie bei allen Katalysatoren müssen auch Mehrkomponentensysteme hinsichtlich Aktivität, Selektivität und Stabilität optimiert werden. Die Skalierbarkeit erfordert zudem reproduzierbare Herstellungsmethoden, die nach wie vor ein kritischer Bereich von Forschung und Entwicklung sind.

Basierend auf dem oben genannten Stand der Technik besteht die Aufgabe der vorliegenden Erfindung darin, einen Katalysator sowie ein mittels des Katalysators betreibbares Verfahren zur Umwandlung von CO₂ unter Verwendung von Wasserstoff bereitzustellen, welcher bzw. welches eine gegenüber dem Stand der Technik verbesserte katalytische Aktivität ermöglicht.

Diese Aufgabe wird durch den Gegenstand der unabhängigen Ansprüche erreicht, wobei weitere vorteilhafte Ausführungsformen in den abhängigen Ansprüchen, Beispielen, Figuren und der allgemeinen Beschreibung der Erfindung beschrieben sind.

### Zusammenfassung der Erfindung

Die erfindungsgemäßen mehrschichtigen Dünnschichtkatalysatoren, die Mehrschichtkatalysatoren, stellen eine verbesserte Umsetzung von Mehrkomponentensystemen dar, bei denen die Komponenten in definierten 3D-Strukturen angeordnet sind. Diese Konfiguration bietet Möglichkeiten, Schnittstellen auf der Nanoskala zu gestalten, Reaktionspfade zu optimieren und die katalytische Effizienz zu verbessern. Die Komponenten sind in der erfinderischen Lösung als Schichten und diese in Bereichen auf einem Substrat angeordnet, wobei die Komponenten jeweils unterschiedliche strukturelle und chemische Eigenschaften aufweisen, die die katalytische Leistung beeinflussen.

Die Integration dieser Prinzipien in 3D-mehrschichtige Dünnschichtkatalysatoren unterstreicht das Potenzial von Mehrkomponentensystemen, die heterogene Katalyse zu revolutionieren. Durch die Nutzung nanoskaliger Präzision und der kooperativen Effekte von Kontaktstellen eignen sich diese Katalysatoren zum Beispiel hervorragend für Anwendungen in der Energieumwandlung und der chemischen Synthese.

Ein erster Aspekt der Erfindung bezieht sich auf einen Mehrschichtkatalysator, aufweisend:
- ein Substrat sowie
- mindestens einen auf dem Substrat angeordneten Stapel, welcher gebildet ist durch mindestens 10 übereinander angeordnete Schichten, wobei benachbarte Schichten unterschiedliche Materialien aufweisen, und wobei der mindestens eine Stapel eine Vielzahl von Aussparungen aufweist, oder auf dem Substrat eine Vielzahl Stapel angeordneten sind,
und wobei der Mehrschichtkatalysator ein Stapeloberfläche-zu-Stapelvolumen-Verhältnis, gebildet aus einer freiliegenden Oberfläche des mindestens einen Stapel oder der Vielzahl von Stapeln zu einem Volumen des mindestens einen Stapel oder der Vielzahl von Stapeln aufweist, das zwischen 100 1/m und 400.000.000 1/m, insbesondere zwischen 10.000 1/m und 4.000.000 1/m beträgt.

Ein zweiter Aspekt der Erfindung bezieht sich auf ein Verfahren zur Umwandlung von CO₂ unter Verwendung von Wasserstoff in Gegenwart eines Mehrschichtkatalysators gemäß dem ersten Aspekt der Erfindung.

### Begriffe und Definitionen

Für die Auslegung dieser Spezifikation gelten die folgenden Definitionen, und wann immer es angebracht ist, beinhalten die im Singular verwendeten Begriffe auch den Plural und umgekehrt. Sollte eine der nachstehenden Definitionen im Widerspruch zu einem Dokument stehen, das durch Verweis in diese Spezifikation aufgenommen wurde, so ist die nachstehende Definition maßgeblich.

Die Begriffe "umfassend", "habend", "enthaltend" und "einschließend" sowie andere ähnliche Formen und ihre grammatikalischen Äquivalente, wie sie hier verwendet werden, sollen in ihrer Bedeutung gleichwertig sein und ein offenes Ende haben, so dass ein Gegenstand oder mehrere Gegenstände, die auf eines dieser Wörter folgen, keine erschöpfende Aufzählung dieses Gegenstands oder dieser Gegenstände darstellen oder nur den oder die aufgeführten Gegenstände beinhalten sollen. Beispielsweise kann ein Artikel, der die Bestandteile A, B und C umfasst, aus den Bestandteilen A, B und C bestehen (d. h. nur diese enthalten) oder nicht nur die Bestandteile A, B und C, sondern auch einen oder mehrere andere Bestandteile enthalten. Es ist beabsichtigt und wird so verstanden, dass "umfasst" und ähnliche Ausführungsformen sowie deren grammatikalische Äquivalente die Offenlegung von Ausführungsformen von "im Wesentlichen bestehend aus" oder "bestehend aus" beinhalten.

Wird ein Wertebereich angegeben, so wird davon ausgegangen, dass jeder Zwischenwert bis zum Zehntel der Einheit des unteren Grenzwerts zwischen dem oberen und dem unteren Grenzwert dieses Bereichs und jedem anderen angegebenen oder dazwischen liegenden Wert in diesem angegebenen Bereich von der Offenbarung umfasst ist, sofern der Kontext nicht eindeutig etwas anderes vorschreibt, vorbehaltlich jedes ausdrücklich ausgeschlossenen Grenzwerts in dem angegebenen Bereich. Beinhaltet der angegebene Bereich einen oder beide Grenzwerte, so sind auch Bereiche, die einen oder beide dieser eingeschlossenen Grenzwerte ausschließen, in der Offenbarung enthalten.

Die Bezugnahme auf "etwa" einen Wert oder Parameter beinhaltet (und beschreibt) Variationen, die sich auf diesen Wert oder Parameter an sich beziehen. Zum Beispiel beinhaltet die Beschreibung von "etwa X" die Beschreibung von "X".

Wie hierin, einschließlich in den beigefügten Ansprüchen, verwendet, beinhalten die Singularformen "ein", "oder" und "die" Pluralreferenzen, sofern der Kontext nicht eindeutig etwas anderes vorschreibt.

### Ausführliche Beschreibung

Ein erster Aspekt der Erfindung betrifft einen Mehrschichtkatalysator, aufweisend ein Substrat sowie mindestens einen auf dem Substrat angeordneten Stapel, welcher aus mindestens 10 übereinander angeordneten Schichten gebildet ist, wobei benachbarte Schichten unterschiedliche Materialien aufweisen, und wobei der Stapel eine Vielzahl von Aussparungen aufweist oder auf dem Substrat eine Vielzahl von Stapeln angeordnet ist. Der Mehrschichtkatalysator weist dabei ein Stapeloberfläche-zu-Stapelvolumen-Verhältnis, welches aus einer freiliegenden Oberfläche des mindestens einen Stapel oder der Vielzahl an Stapeln zu einem Volumen des mindestens einen Stapel oder der Vielzahl an Stapel aufweist, das zwischen 100 1/m und 400.000.000 1/m, insbesondere zwischen 10.000 1/m und 4.000.000 1/m beträgt.

Die freiliegenden Oberflächen eines Stapels sind als die Oberflächen des Stapels zu verstehen, die nicht in direktem Kontakt zu anderen Strukturen stehen, beispielsweise zum Substrat, und entsprechend katalytisch aktiv sein können, da diese Flächen für gasförmige oder flüssige Medien erreichbar sind.

Die Erfindung offenbart zwei Varianten, die derartige Stapeloberfläche-zu-Stapelvolumen-Verhältnisse des Mehrschichtkatalysators bzw. des erfindungsgemäßen mindestens einen Stapel zu optimieren: Einerseits kann ein erfindungsgemäßer Stapel, insbesondere ein einziger solcher Stapel, auf dem Substrat eine Vielzahl von Aussparungen aufweisen, welche entsprechend zu einem erhöhten Oberfläche-zu-Volumen-Verhältnis führen. Andererseits können auch eine Vielzahl von erfindungsgemäßen Stapeln auf dem Substrat angeordnet sein, was ebenfalls das Oberfläche-zu-Volumen-Verhältnis der Stapel des Mehrschichtkatalysators erhöht.

Nach einer weiteren Ausführungsform der Erfindung ist ein Material des Substrats aus Glas, Keramik, Metall ausgewählt oder das Substrat generell aus einem kristallinen Material gefertigt. Eine Ausführung der Substrate aus Kunststoff, die auch flexibel sein können, ist für bestimmte Anwendungen auch gegeben.

Nach einer Ausführungsform der Erfindung umfasst der mindestens eine Stapel mindestens 20 Schichten.

In bestimmten Ausführungsformen umfasst der mindestens eine Stapel im Mehrschichtkatalysator mindestens 50 Schichten.

In bestimmten Ausführungsformen umfasst der Mehrschichtkatalysator mindestens 100 Schichten.

Nach einer Ausführungsform der Erfindung umfasst der Stapel 10 bis 1000 Schichten, insbesondere 10 bis 200 Schichten, spezieller 20 bis 200 Schichten, noch spezieller 50 bis 200 Schichten.

Nach einer Ausführungsform der Erfindung umfasst der Stapel 10 bis 1000 Schichten, insbesondere 50 bis 800 Schichten, speziell 100 bis 800 Schichten, noch spezieller 100 bis 500 Schichten, insbesondere 250 bis 500 Schichten.

In bestimmten Ausführungsformen umfasst der Mehrschichtkatalysator 10 bis 1000 Schichten.

In bestimmten Ausführungsformen umfasst der Mehrschichtkatalysator 10 bis 200 Schichten.

In bestimmten Ausführungsformen umfasst der Mehrschichtkatalysator 20 bis 200 Schichten.

In bestimmten Ausführungsformen umfasst der Mehrschichtkatalysator 50 bis 200 Schichten.

In bestimmten Ausführungsformen umfasst der Mehrschichtkatalysator 10 bis 1000 Schichten.

In bestimmten Ausführungsformen umfasst der Mehrschichtkatalysator 50 bis 800 Schichten.

In bestimmten Ausführungsformen umfasst der Mehrschichtkatalysator 100 bis 800 Schichten.

In bestimmten Ausführungsformen umfasst der Mehrschichtkatalysator 100 bis 500 Schichten.

Nach einer weiteren Ausführungsform der Erfindung weist jede der Schichten eine Schichtdicke im Bereich von einer Atomlage bis 50 nm auf.

In einer Ausführungsform weist jede der Schichten eine Schichtdicke im Bereich von 0,5 nm bis 30 nm auf.

In einer Ausführungsform weist jede der Schichten eine Schichtdicke im Bereich von 1 nm bis 10 nm.

In einer Ausführungsform weist jede der Schichten eine Schichtdicke im Bereich von 10 nm bis 30 nm aufweist.

Nach einer weiteren Ausführungsform der Erfindung sind die Materialien der Schichten aus Metallen; Metalloxiden; Metall- oder Kohlenstoffnitriden; Metallcarbiden; Legierungen; insbesondere aus Metallen und Metalloxiden ausgewählt.

Nach einer weiteren Ausführungsform der Erfindung sind die Materialien der Schichten aus Metalloxid und Metall ausgewählt.

Nach einer weiteren Ausführungsform der Erfindung weist jede Schicht eine Schichtdicke auf, die innerhalb eines durch das Material der Schicht definierten Schichtdickenbands liegt. Zum Beispiel kann der Stapel Schichten aufweisen, die als Diffusionsbarriere fungieren, um ein Verschmelzen der Schichten zu verhindern. Derartige Schichten sind vorzugsweise wesentlich dünner als katalytisch aktive Schichten, insbesondere mindestens zehnmal dünner als katalytisch aktive Schichten.

Nach einer weiteren Ausführungsform der Erfindung beträgt eine Höhe des Stapels 0,5 µm bis 30 µm. Die Höhe des Stapels wird dabei verstanden als eine maximale Erstreckung des Stapels entlang einer Richtung, die orthogonal zu einer Oberfläche des Substrats ist.

Nach einer weiteren Ausführungsform der Erfindung beträgt die Höhe des Stapels 1 µm bis 30 µm.

Nach einer weiteren Ausführungsform der Erfindung beträgt die Höhe des Stapels 5 µm bis 30 µm.

Nach einer weiteren Ausführungsform der Erfindung beträgt die Höhe des Stapels 10 µm bis 30 µm.

Nach einer weiteren Ausführungsform der Erfindung sind die Materialien der Schichten aus Metalloxiden ausgewählt.

Nach einer weiteren Ausführungsform der Erfindung folgt auf eine Metallschicht mindestens eine Metalloxidschicht.

Nach einer weiteren Ausführungsform der Erfindung folgen auf eine Metallschicht mindestens zwei Metalloxidschichten.

Nach einer weiteren Ausführungsform der Erfindung folgen auf eine Metallschicht mindestens drei Metalloxidschichten.

Nach einer weiteren Ausführungsform der Erfindung ist das Metall der Metallschicht aus drei verschiedenen Metallen, insbesondere zwei verschiedenen Metallen, ausgewählt, wobei insbesondere das Metall der Metallschicht immer dasselbe ist.

Nach einer weiteren Ausführungsform der Erfindung ist das Metalloxid der Metalloxidschicht aus sechs verschiedenen Metalloxiden, insbesondere drei verschiedenen Metallen, ausgewählt.

Nach einer weiteren Ausführungsform der Erfindung ist der Mehrschichtkatalysator, insbesondere jeder Stapel des Mehrschichtkatalysators, unter anderem dazu ausgebildet, ausgewählte Reaktanden, insbesondere Wasserstoff und/oder Kohlendioxid, zu aktivieren. Wasserstoff und Kohlenstoff kann dabei selektiv von bestimmten Schichten des Mehrschichtkatalysators aktiviert werden. Insbesondere sind z.B. in einem Cu-ZnO Mehrschichtkatalysator Cu-Schichten bzw. deren freiliegende Oberflächen dazu ausgebildet, Wasserstoff zu aktivieren und ZnO-Schichten bzw. deren freiliegende Oberflächen sind dazu ausgebildet, CO₂ zu aktivieren. Der dissoziierte Wasserstoff ist auf der freiliegenden Oberfläche des Mehrschichtkatalysators mobil und kann sich zum aktivierten CO₂ am ZnO bewegen.

Jedes Material der Schicht erfüllt eine eigene Funktion, wie beispielsweise die oben genannte Aktivierung als auch eine mögliche Diffusionsbarriere, um ein Vermischen der Schichten, z.B. während der Präparation derselben, zu verhindern.

Nach einer weiteren Ausführungsform der Erfindung weisen die Schichten Metallschichten und Metalloxidschichten auf, wobei im Stapel jeweils eine Metallschicht und eine Metalloxidschicht abwechselnd übereinander angeordnet sind.

Nach einer weiteren Ausführungsform der Erfindung sind die Schichten Metalloxidschichten.

Nach einer weiteren Ausführungsform der Erfindung weisen die Schichten Metallschichten und Metallcarbidschichten auf, wobei im Stapel jeweils eine Metallschicht und eine Metallcarbidschicht abwechselnd übereinander angeordnet sind.

Nach einer weiteren Ausführungsform der Erfindung weisen die Schichten Metallschichten und Metallnitridschichten auf, wobei im Stapel jeweils eine Metallschicht und eine Metallnitridschicht abwechselnd übereinander angeordnet sind.

Nach einer Ausführungsform der Erfindung umfasst der Mehrschichtkatalysator mindestens zwei Schichten, die eine Metallschicht und eine Metalloxidschicht sind.

Nach einer Ausführungsform der Erfindung wechseln sich die Metallschicht und eine Metalloxidschicht ab.

Nach einer Ausführungsform der Erfindung enthält die Metallschicht Kupfer oder besteht aus Kupfer, wobei die Metalloxidschicht Zinkoxid enthält oder aus Zinkoxid besteht.

Nach einer Ausführungsform der Erfindung umfasst der Mehrschichtkatalysator mindestens zwei Schichten, die Metallschichten sind.

Nach einer Ausführungsform der Erfindung umfasst der Mehrschichtkatalysator mindestens zwei Schichten, die Metalloxidschichten sind.

Nach einer Ausführungsform der Erfindung umfasst der Mehrschichtkatalysator mindestens zwei Schichten, die eine Metallschicht und eine Metallcarbidschicht sind.

Nach einer Ausführungsform der Erfindung wechseln sich die Metallschicht und eine Metallcarbidschicht ab.

Nach einer Ausführungsform der Erfindung umfasst der Mehrschichtkatalysator mindestens zwei Schichten, die eine Metallschicht und eine Metallnitridschicht sind.

Nach einer Ausführungsform der Erfindung wechseln sich die Metallschicht und eine Metallnitridschicht entlang der Stapelrichtung ab.

Nach einer weiteren Ausführungsform der Erfindung ist das Metall ein Übergangsmetall oder eine Legierung.

Nach einer weiteren Ausführungsform der Erfindung ist das Metall aus der Gruppe Fe, Co, Ni, Cu, Ru, Rh, Pd, Pt, Re, Au, Ag, Ir und Kombinationen davon, insbesondere Fe, Co, Ni, Cu, Rh, Pd, Pt, ausgewählt.

Nach einer weiteren Ausführungsform der Erfindung ist das Metalloxid aus einer der Gruppen Lanthanoidoxide, Erdalkalimetalloxide, Übergangsmetalloxide, Siliziumoxide und Oxide der Borgruppe und Kombinationen davon ausgewählt.

Nach einer weiteren Ausführungsform der Erfindung ist das Metalloxid aus einer der folgenden Gruppen ausgewählt:
a. Magnesiumoxid, Calciumoxid, Strontiumoxid, Bariumoxid,
b. Aluminiumoxid, Boroxid, Galliumoxid, Indiumoxid,
c. Siliziumoxid,
d. Titanoxid, Vanadiumoxid, Chromoxid, Manganoxid, Eisenoxid, Nickeloxid, Kobaltoxid, Kupferoxid, Zinkoxid,
e. Zirkoniumoxid, Molybdänoxid, Rheniumoxid,
f. Lanthanoxid, Ceroxid, Samariumoxid.

Nach einer weiteren Ausführungsform der Erfindung ist das Metallcarbid ein Übergangsmetallcarbid, insbesondere ein Übergangsmetallcarbid, das aus Mo, W und Fe ausgewählt ist.

Nach einer weiteren Ausführungsform der Erfindung ist das Metallnitrid ein Übergangsmetallnitrid, insbesondere ein Übergangsmetallnitrid, ausgewählt aus Mo, W und Fe, oder ein Siliziumnitrid.

Nach einer weiteren Ausführungsform der Erfindung weist der Stapel in einer Querschnittsebene, die sich senkrecht zu einer Stapelrichtung erstreckt, entlang der die Schichten übereinander angeordnet sind, eine Querschnittsfläche auf, die im Wesentlichen rechteckig oder im Wesentlichen elliptisch ist.

Nach einer weiteren Ausführungsform der Erfindung weist die Vielzahl von Stapeln auf dem Substrat eine Stapeldichte zwischen 10.000/cm² und 10.000.000.000/cm² auf.

Hierbei kann die Höhe der Stapel insbesondere zwischen 0,5 µm und 30 µm liegen. Die Kombination aus einer hohen Stapeldichte auf dem Substrat bei einer relativ geringen Höhe der Stapel resultiert in einem geringen Volumen der einzelnen Stapel und einem entsprechend hohen Stapeloberflächen-zu-Stapelvolumen-Verhältnis des Mehrschichtkatalysators bzw. seiner Stapel.

Nach einer Ausführungsform der Erfindung weist der Mehrschichtkatalysator 1 bis 400 x 10⁹ Stapel auf einer 1 cm² großen Oberfläche des Substrats auf.

Insbesondere weist wenigstens ein Stapel, speziell mehrere Stapel, noch spezieller jeder Stapel der Vielzahl von Stapeln, eine im Wesentlichen rechteckige, als Grenzfall auch quadratische oder im Wesentlichen elliptische, als Grenzfall auch kreisförmige Querschnittsfläche auf.

Nach einer weiteren Ausführungsform der Erfindung weist die Vielzahl von Aussparungen des mindestens einen Stapel auf dem Substrat eine Aussparungsdichte zwischen 10.000/cm² und 10.000.000.000/cm² auf.

Insbesondere ist mindestens eine Aussparung zumindest abschnittsweise entlang der Stapelrichtung ausgebildet.

Insbesondere bildet mindestens eine Aussparung, insbesondere jede der Vielzahl von Aussparungen, ein Durchgangsloch durch den Stapel. Das jeweilige Durchgangsloch erstreckt sich dabei vorzugsweise nicht in das Substrat hinein, sodass durch ein Durchgangsloch durch den Stapel höchstens eine Oberfläche des Substrats freigelegt ist.

Alternativ oder zusätzlich kann mindestens eine Aussparung ein Sackloch an dem Stapel bilden.

Der Mehrschichtkatalysator kann mehrere Stapel aufweisen, wobei wenigstens ein Stapel, speziell mehrere Stapel, noch spezieller jeder Stapel wenigstens eine Aussparung aufweist. Bei der Aussparung oder den Aussparungen kann es sich dabei um Durchgangslöcher und/oder Sacklöcher handeln.

Insbesondere weist eine Vielzahl von auf dem Substrat angeordneten Stapeln jeweils eine Vielzahl von Aussparungen auf. Dadurch wird das Stapeloberfläche-zu-Stapelvolumen-Verhältnis einerseits durch die Vielzahl von auf dem Substrat angeordneten Stapel erhöht. Andererseits erhöht sich das Stapeloberfläche-zu-Stapelvolumen-Verhältnis durch die Ausprägung der Vielzahl von Aussparungen innerhalb der einzelnen Stapel weiter. Mit anderen Worten werden hierbei die beiden erfindungsgemäßen Varianten von Stapeln mit einer Vielzahl von Aussparungen und einer Vielzahl von Stapeln miteinander kombiniert.

Nach einer weiteren Ausführungsform der Erfindung weist jeder Stapel der Vielzahl von Stapeln wenigstens ein Paar einander gegenüberliegender freiliegender Oberflächenabschnitte auf, wobei ein maximaler Abstand zwischen dem Paar der gegenüberliegenden freiliegenden Oberflächenabschnitten im Bereich von 1 nm bis 50 µm liegt, insbesondere wobei der Abstand zwischen den zwei gegenüberliegenden freiliegenden Oberflächenabschnitten im Bereich von 10 nm bis 50 µm liegt, speziell wobei der Abstand zwischen den zwei offenen Oberflächenabschnitten im Bereich von 1 µm bis 50 µm liegt. Die gegenüberliegenden freiliegenden Oberflächenabschnitte können beispielsweise zwei Seitenflächen eines Stapels mit einem im Wesentlichen rechteckigen Querschnitt bilden. Bei elliptischen oder kreisförmigen Querschnitten der Stapel ohne Aussparungen kann der Abstand zwischen den beiden gegenüberliegenden freiliegenden Oberflächenabschnitten eines Stapels auch als ein Durchmesser des Stapels verstanden werden.

Alternativ oder zusätzlich kann der Abstand zwischen den zwei gegenüberliegenden freiliegenden Oberflächenabschnitten einen Durchmesser einer Aussparung eines Stapels bezeichnen, insbesondere eines Durchgangslochs. Wird ein Mehrschichtkatalysator mit einem Stapel mit insbesondere einer Vielzahl von Aussparungen verwendet, kann sich also der Durchmesser der Aussparungen in derselben Größenordnung bewegen wie ein Durchmesser einzelner Stapel bei einem Mehrschichtkatalysator mit einer Vielzahl von Stapeln.

Ein zweiter Aspekt der Erfindung betrifft ein Verfahren zur Umwandlung von CO₂ unter Verwendung von Wasserstoff in Gegenwart eines Mehrschichtkatalysators gemäß dem ersten Aspekt der Erfindung.

Sofern nicht anders definiert, haben alle hierin verwendeten technischen und wissenschaftlichen Begriffe die gleiche Bedeutung, wie sie in der Fachwelt allgemein üblich ist.

Die Erfindung wird durch die folgenden Beispiele weiter veranschaulicht, aus denen sich weitere Ausführungsformen und Vorteile ergeben. Diese Beispiele dienen der Veranschaulichung der Erfindung, beschränken aber nicht ihren Umfang.

### Beschreibung der Abbildungen

- Fig. 1: zeigt ein Schema einer Sputtervorrichtung für die Herstellung eines erfindungsgemäßen Mehrschichtkatalysators A, sowie schematisch einen auf einem Glassubstrat abgeschiedenen Mehrschichtkatalysator mit einem einzigen Stapel übereinander angeordneter Schichten B.
- Fig. 2: zeigt A einen Mehrschichtkatalysator mit einem einzigen Stapel und B das Schema des Strukturierungsprozesses dieses Stapels mit einem gepulsten Laser, zur Herstellung eines Mehrschichtkatalysators mit einer Vielzahl von Stapeln.

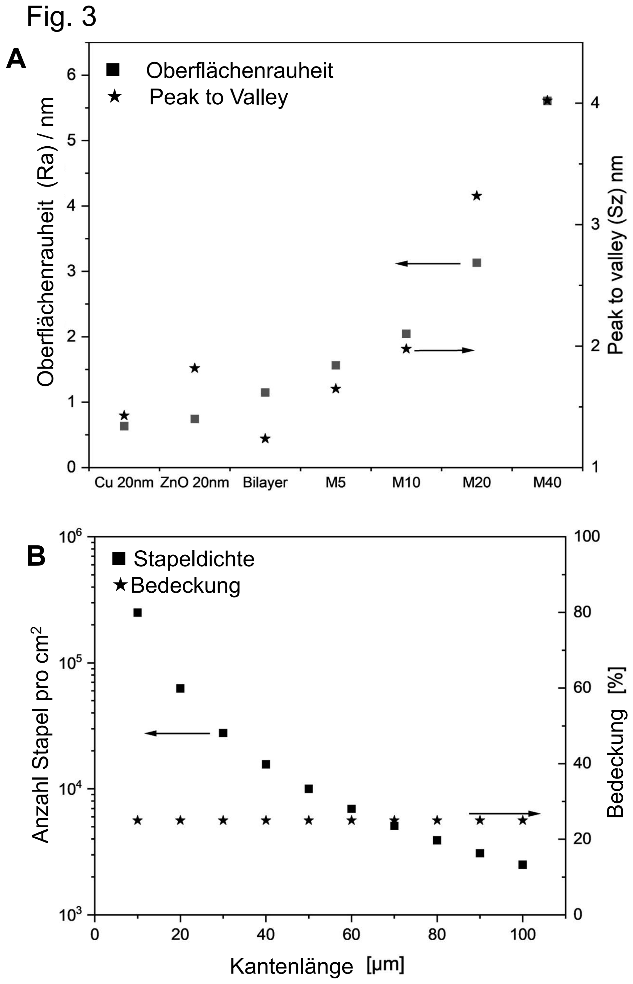

- Fig. 3: zeigt A die Oberflächenrauheit zusammen mit der "Peak to Valley" Größe für den Aspekt der Rauheit eines erfindungsgemäßen Mehrschichtkatalysators für verschiedene Anzahlen von Schichten, wobei M5 für 5 alternierende Schichten, M10 für 10 alternierende Schichten, M20 für 20 alternierende Schichten und M40 für 40 alternierende Schichten steht. B zeigt die Stapeldichte eines erfindungsgemäßen Mehrschichtkatalysators in Abhängigkeit von der Kantenlänge der einzelnen Stapel, wobei die quadratische Korrelation zwischen Größenreduktion und Schnittstellendichte hervorgehoben wird. Die Bedeckung ist in B ebenfalls aufgetragen

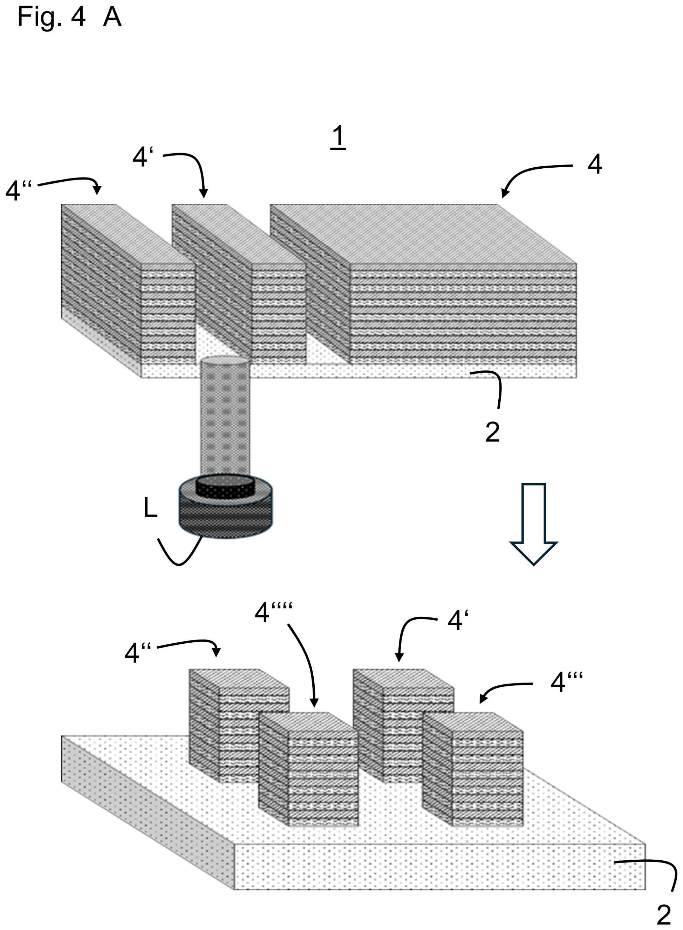

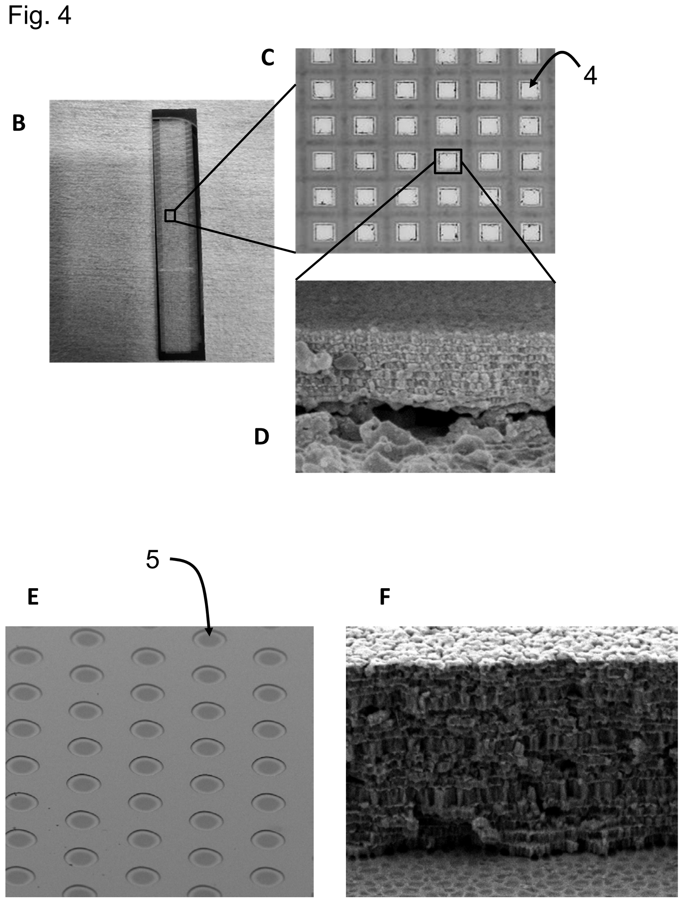

- Fig. 4: zeigt A die Strukturierung einer Vielzahl von Stapeln des Mehrschichtkatalysators durch gepulste Laserablation zu Stapeln mit quadratischer Querschnittsfläche, B eine optische Aufnahme eines strukturierten Kupfer-Zinkoxid-Mehrschichtkatalysators auf einem Glassubstrat, C eine mikroskopische Aufnahme des I strukturierten Kupfer-Zinkoxid-Mehrschichtkatalysators auf dem Glassubstrat, D eine REM-Aufnahme einer freiliegenden Oberfläche des Kupfer-Zinkoxid-Mehrschichtkatalysators nach der Laserablation, E ein REM-Aufnahme eines Stapels eines Mehrschichtkatalysators mit einer Vielzahl von kreisförmigen Aussparungen sowie F eine REM-Aufnahme einer resultierenden freiliegenden Oberfläche.
- Fig. 5: zeigt die Reaktionsbedingungen für die katalytische Prüfung eines lasergravierten CuZnO-Mehrschichtkatalysators. Reaktionsdruck: 30 bar.
- Fig. 6: zeigt die Gaschromatographie-Analyse eines CuZnO-Mehrschichtkatalysators mit 8 Substraten (Anzahl der Schichten = 200), abgeschieden auf Corning-Glas mit den Abmessungen 10 x 7,5 mm. (Schwarz) CO₂-Umwandlung (%) bei unterschiedlichen Reaktionstemperaturen und (Grau) Produktselektivität (%) für Methanol, Methan und CO bei unterschiedlichen Reaktionstemperaturen..
- Fig. 7: zeigt A eine REM-Aufnahme eines FIB-Querschnitts eines erfindungsgemäßen Kupfer-Zinkoxid-Mehrschichtkatalysators mit zweimal 20 Schichten im Stapel vor und B nach 3 Tagen kontinuierlicher CO₂-Hydrierung bei 240°C, 40 bar und einem Gasverhältnis von 3:1:1 von H₂:CO₂:Ar.

### Beschreibung der Tabellen

- Tabelle 1: zeigt die Parameter und Fähigkeiten des Lasersystems.
- Tabelle 2: zeigt die Ergebnisse der Gaschromatographie zur CO₂-Umwandlung (%) bei unterschiedlichen Reaktionstemperaturen.
- Tabelle 3: zeigt die Ergebnisse der Gaschromatographie zur Produktselektivität (%) für Methanol und CO bei unterschiedlichen Reaktionstemperaturen.

### Beispiele

### Beispiel 1: Herstellung eines 3D-strukturierten, Kupfer-Zinkoxid-Mehrschichtkatalysator als CO/CO₂-Hydrierungskatalysator

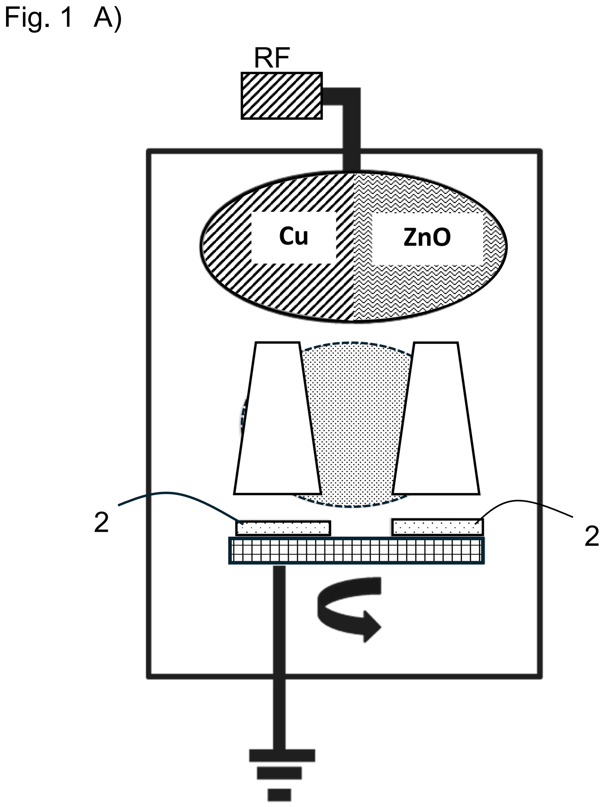

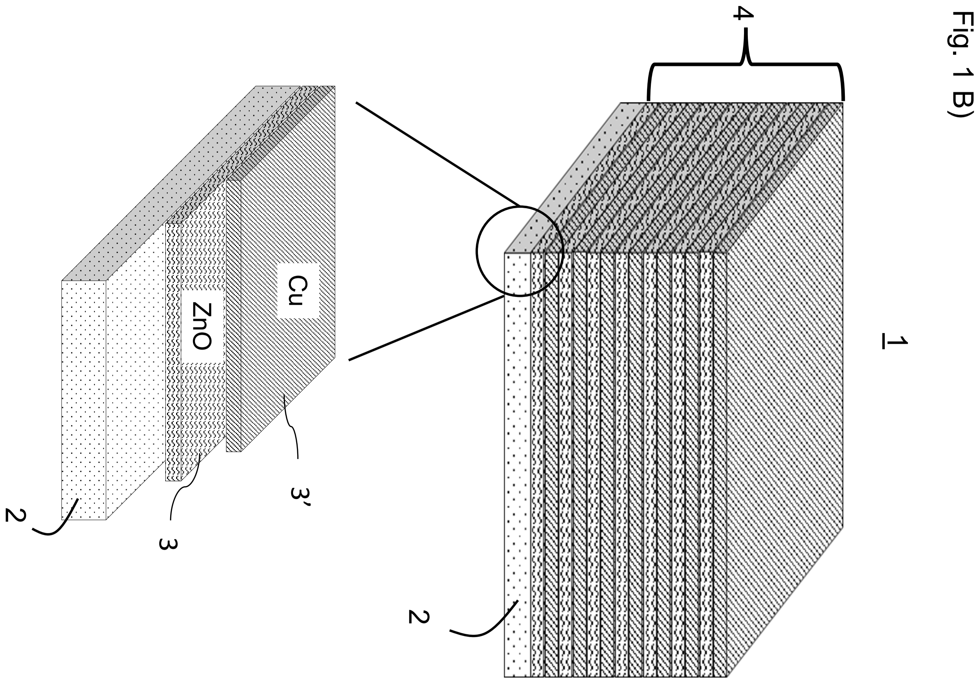

Ein dreidimensionaler -Mehrschichtkatalysator 1, mit alternierenden Schichten von Kupfer (Cu) und Zinkoxid (ZnO), die einen Stapel 4 bilden, wurde als Hydrierungskatalysator hergestellt. Die iterative Abscheidung von Cu- und ZnO-Schichten wurde auf einem Substrat 2 aus Glas unter Verwendung von Hochfrequenzsputtern durchgeführt (Fig.1 A und B). In der Fig. 1 B ist aus dem Stapel 4 ein Ausschnitt herausvergrößert, der exemplarisch für die Vielzahl Schichten im Stapel 4 je eine Schicht 3 ZnO und eine Schicht 3' Cu auf dem Substrat 2 veranschaulicht. Die Vorrichtung zum Hochfrequenzsputtern, wie sie in der Fig. 1A gezeigt ist, dient dem sogenannten Co-Sputtern. Dabei wird die Abscheidung der zwei unterschiedlichen Schichten durch mehrere Sputter-Targets (Cu und ZnO) erzielt, die abwechselnd auf rotierende Substrate 2 abscheiden.

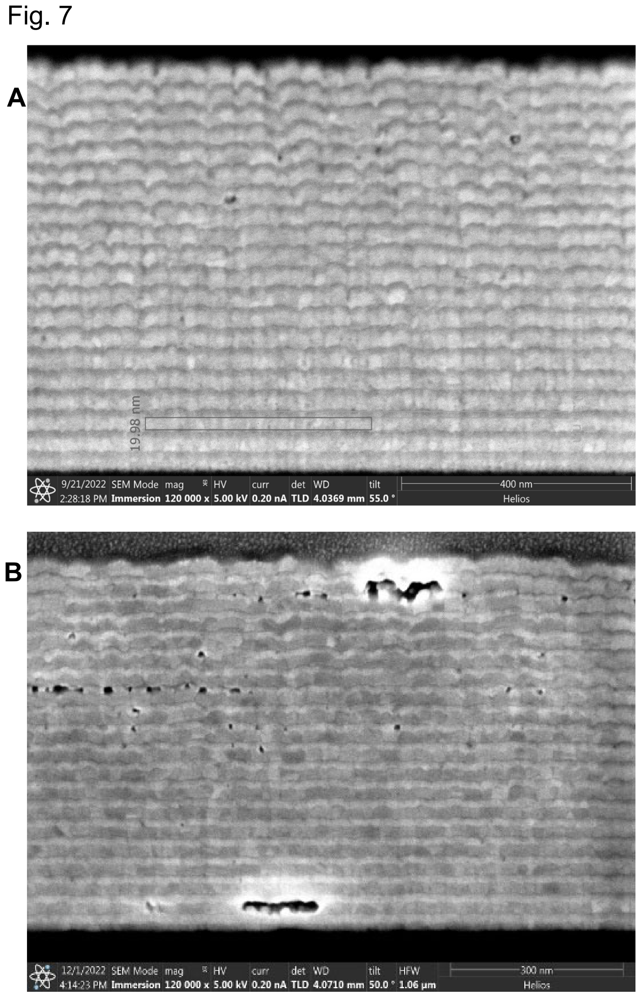

Die Schichtdicken im 20 Schichten aufweisenden Stapel 4 des Mehrschichtkatalysators 1 aus alternierenden Schichten von Cu und ZnO wurde im Durchschnitt mit 19,98 nm für die Kupferschicht und 10,34 nm für die Zinkoxidschicht gemessen. Die Schichten sind in Fig. 7A) durch eine REM-Aufnahme eines mit FIB (engl.: Focused Ion Beam) hergestellten Querschnitts eines Stapels 4, eines erfindungsgemäßen Kupfer-Zinkoxid-Mehrschichtkatalysators 1 mit zweimal 20 Schichten im Stapel 4, abgebildet. Die Schichtdicke jeder Cu- und ZnO-Schicht (3, 3') kann präzise kontrolliert werden, um eine optimale katalytische Aktivität an der Cu:ZnO-Schnittstelle sicherzustellen.

Die Oberflächenrauheit nimmt mit der Anzahl der Schichten im Stapel 4 zu, von M5 (Ra = 1,5 nm) bis M40 (Ra = 3,5 nm), wie in Fig. 3A gezeigt. Gleichzeitig nimmt der "Peak to Valley" Aspekt für mehrere Schichten im Stapel (M5 - M40) auch zu. Dieses Verhalten spiegelt den kumulativen Effekt alternierender Abscheidung und Schnittstelleninteraktionen über mehrere Schichten im Stapel 4 wider.

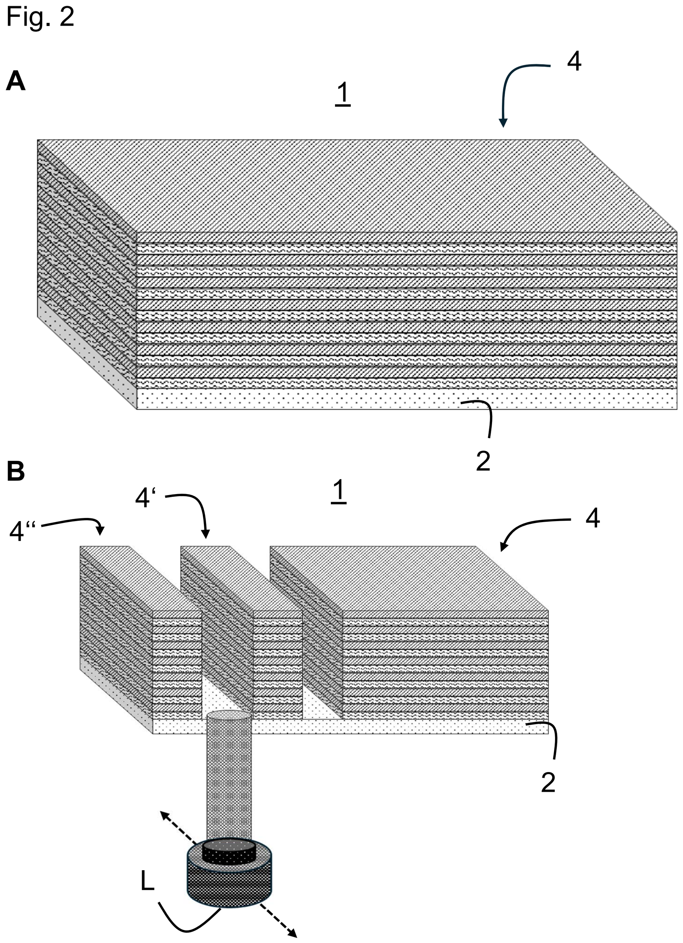

Eine Laserablationstechnik L mit den spezifizierten Einstellungen (Fig. 2 A und B, Tabelle 1) wurde angewendet, um gezielt dünne Funktionsschichten zu entfernen und die Herstellung von 3D-strukturierten Mehrschichtkatalysatoren 1 mit einer Vielzahl von Stapeln 4', 4", 4‴, 4"" zu ermöglichen, wie in Fig. 2 B sowie insbesondere in Fig. 4 A dargestellt ist. Die Vielzahl von Stapeln 4', 4", 4‴, 4"" und die geringen Abmessungen bzw. Volumina der einzelnen Stapel 4 ermöglichen vorteilhaft erhöhte Stapeloberflächen-zu-Stapelvolumen-Verhältnisse des Mehrschichtkatalysators 1 bzw. seiner Stapel 4. Alternativ oder zusätzlich zu einer Vielzahl von Stapeln 4', 4", 4"', 4"" kann der Mehrschichtkatalysator 1 auch zumindest einen Stapel 4 mit einer Vielzahl an Aussparungen 5 aufweisen, die insbesondere Durchgangslöcher durch den Stapel 4 bilden können, wie in Fig. 4 E beispielshaft mit Durchgangslöchern mit kreisförmigem Querschnitt gezeigt ist, welche das, unter dem Stapel 4 liegende Substrat freilegen. Auch auf diese Weise lässt sich das Stapeloberflächen-zu-Stapelvolumen-Verhältnis des Mehrschichtkatalysators 1 wesentlich erhöhen, da die Durchgangslöcher mittels Laserablationstechnik L präzise und mit einer hohen Aussparungsdichte hergestellt werden können.

**Tabelle 1**

| **Parameter** | **ns-Laser** | **ps-Laser** |
|---|---|---|
| **Wellenlänge (nm)** | 532 | 355, 532, 1064 |
| **Pulsbreite** | 12 ns | 12 ps |
| **Wiederholrate PRR (kHz)** | 20-400 | 50-8200 |
| **Maximale Pulsenergie (µJ)** | 41 | 60, 67, 96 |
| **Puls-Spitzenleistung (kW)** | 2.7 | 5000, 5583, 8000 |
| **M²** | <1.2 | <1.3 |
| **Strahltaille (µm)** | 25, 20 | 20, 21.5 |
| **Rayleigh-Länge (mm)** | 3.8, 3.5 | 2.4, 1.4 |
| **Fokustiefe (mm)** | 7.6, 7.0 | 4.8, 2.8 |

Dieser Prozess erhöht die freiliegende Oberfläche einzelner Stapel 4 des Mehrschichtkatalysators 1 und verbessert die Umwandlungsraten. Die Laserparameter wurden angepasst, so dass die Spotgröße zwischen 20-80 µm lag, bei einer Positioniergenauigkeit von <5 µm. Rasterelektronenmikroskopie (REM)-Bilder der abgeschiedenen Mehrschichtkatalysators 1 wurden sowohl vor als auch nach dem Laserabtrag aufgenommen (Fig. 7 A, vor, und B, nach, und Fig. 4 F und D nach Laserabtrag).

Die Dichte des 3D-strukturierten Stapel 4 des Mehrschichtkatalysators 1 wurde in Abhängigkeit von der Laserstrahlbreite bewertet. Die Laserparameter wurden variiert, um Kantenlängen einzelner Stapel 4 des Mehrschichtkatalysators 1 zwischen 50 µm und 100 µm zu erzeugen (Fig. 3 B). Die einzelnen Stapel 4 wiesen in diesem Beispiel dabei eine quadratische Querschnittsfläche parallel zur darunterliegenden Oberfläche des Substrats 2 auf. Die Dichte der abgetragenen Stapel 4 bzw. Quadrate pro cm² Substratoberfläche nimmt mit zunehmender Kantenlänge des Stapels 4 ab, wobei ein inverser Trend besteht. Bei einer Kantenlänge von 50 µm erreichte die Dichte 10⁴ Quadrate/cm², während sie bei 100 µm auf 2 × 10³ Quadrate/cm² sank. Die Bedeckung blieb dabei konstant. Die Kantenlänge eines Stapels 4 kann dabei als ein Abstand zwischen einem Paar einander gegenüberliegender freiliegender Oberflächenabschnitte eines Stapels 4', 4", 4"', 4ʺʺ definiert werden, wie sie beispielsweise in Fig. 4 A eingezeichnet sind. Die hohe Stapeldichte aufgrund der kurzen Kantenlänge und geringen Volumina der einzelnen Stapel 4 führt zu einem entsprechend hohen Stapeloberfläche-zu-Stapelvolumen-Verhältnis, was die katalytische Aktivität des Mehrschichtkatalysators 1 erhöht.

In den Fig. 4 B, C D sind in B eine optische Aufnahme eines, in einem Teilabschnitt, strukturierten Kupfer-Zinkoxid-Mehrschichtkatalysators auf einem Glassubstrat gezeigt und in C eine mikroskopische Aufnahme des strukturierten Kupfer-Zinkoxid-Mehrschichtkatalysators auf dem Glassubstrat, in der die Stapel 4 zu erkennen sind und in D ist eine REM-Aufnahme einer freiliegenden Oberfläche nach der Laserablation eines Stapels im Kupfer-Zinkoxid-Mehrschichtkatalysators abgebildet. (Es ist der Übersichtlichkeit halber nur ein Stapel 4 mit einem Bezugszeichen versehen.) Ein REM-Aufnahme eines Stapels eines Mehrschichtkatalysators mit einer Vielzahl von kreisförmigen Aussparungen 5 ist in E sowie in F eine REM-Aufnahme einer freiliegenden Oberfläche in einer kreisförmigen Aussparung 5 gezeigt. (Es ist der Übersichtlichkeit halber nur eine Aussparung 5 mit einem Bezugszeichen versehen.)

Der prozentuale Anteil der durch die Laserablation erzeugten Fläche blieb stabil. Diese Ergebnisse unterstreichen die quadratische Korrelation zwischen Größenreduktion und Schnittstellendichte.

### Beispiel 2: Katalytische Prüfung des Cu/ZnO-Mehrschichtkatalysators für CO/CO₂

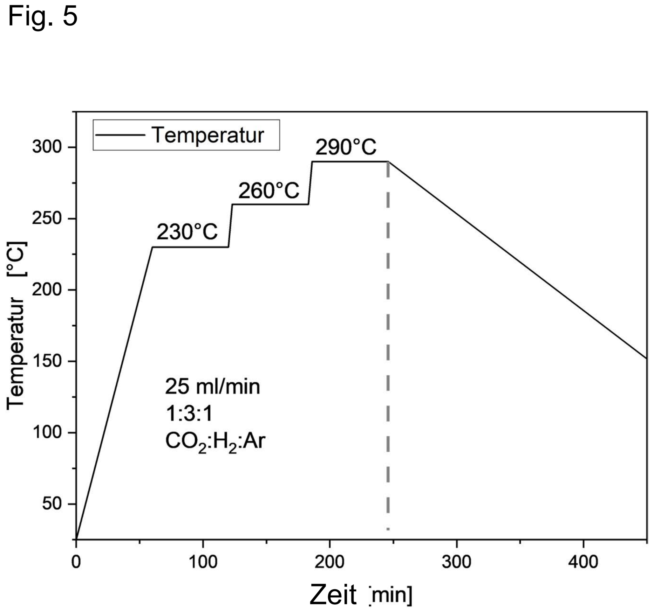

Der Mehrschichtkatalysator 1 wurde in einem kommerziellen Berty Reaktor montiert und ein Gasgemisch aus CO₂, H₂ und Ar im Verhältnis 1:3:1 wurde unter kontrollierten Druck- und Temperaturbedingungen in den Reaktor eingeleitet. Die Gesamtdurchflussrate des Gasgemischs wurde auf 25 mL/min gehalten, mit individuellen Durchflussraten von 5 mL/min für CO₂ und 15 mL/min für H₂ (Fig. 5).

Die Reaktion wurde bei 230 °C gestartet, indem die Temperatur in schrittweisen Erhöhungen von 30 °C bei einer Aufheizrate von 10 °C/min bis zu einem Maximum von 290 °C angehoben wurde. Um einen kontrollierten Abkühlungsprozess zu gewährleisten, wurde am Ende Argon-Gas in das System eingeführt, um die Anlage effizient zu spülen.

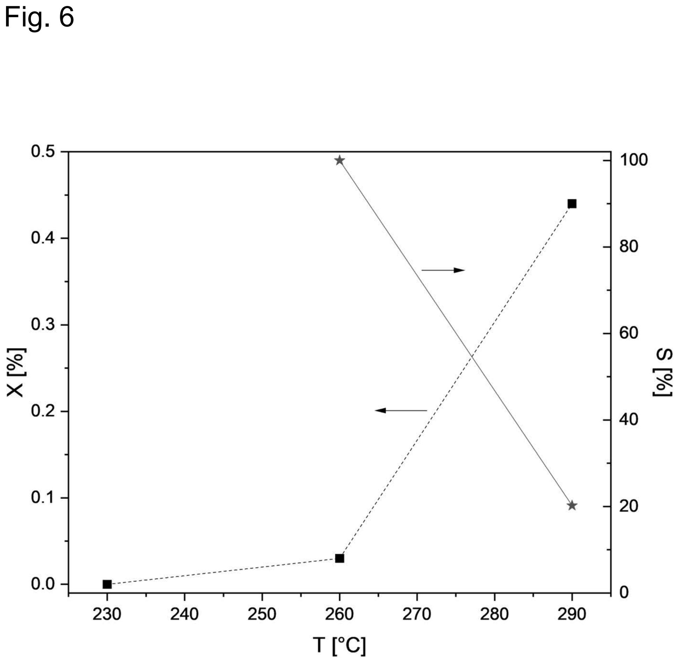

Die Umwandlungsrate und die Produktselektivitätsprozente wurden mittels Gaschromatographie gemessen. Die Gaschromatographie-Analyse zeigte CO₂-Umsätze (X) von bis zu 0.4 % bei 290 °C (Fig. 6, Tabelle 2), wobei die Selektivität (S) bei 260 °C maximal war (Fig. 6, Tabelle 3).

**Tabelle 2**

| **Temperatur (°C)** | **230** | **260** | **290** |
|---|---|---|---|
| CO₂-Umwandlung (%) | 0 | 0,03 | 0,44 |

**Tabelle 3**

| **Temperatur [°C]** | **CO Selektivität [%]** | **CH₃OH Selektivität [%]** |
|---|---|---|
| **230** | - | - |
| **260** | Unter Bestimmungsrenze | 100 |
| **290** | 80 | 20 |

### Beispiel 3: Materialien und Methoden

### Substratvorbereitung

Ein geeignetes Substrat 2 des Mehrschichtkatalysators 1, wie beispielsweise ein Siliziumwafer oder Glas, wurde gereinigt und behandelt, um ein homogenes Wachstum der Schichten 4 zu gewährleisten.

### Katalysatorherstellung

Die Herstellung der Schichten in dem Mehrschichtkatalysator kann mit allen Methoden erfolgen, mit denen sich feste, d.h. amorphe, kristalline oder polykristalline Filme herstellen lassen. Bekannte Methoden sind z.B. die physikalische Gasphasenabscheidung (engl.: physical vapour deposition, kurz PVD), wie diese exemplarisch in dem Übersichtsartikel von K. Reichelt und X. Jiang (The preparation of thin films by physical vapor deposition, Thin Solid Films, Vol. 191, 1990, S. 91-126) beschrieben ist oder auch die chemische Gasphasenabscheidung (engl.: chemical vapour deposition, CVD) wie diese in dem Übersichtsartikel von A. Sherman (Plasma-assisted chemical vapor deposition processes and their semiconducter applications, Thin Solid Films, Vol. 113, 1984, S. 135-149) vorgestellt ist. Das sogenannte "Sputtern" ist eine Methode der physikalischen Gasphasenabscheidung und wird z.B. in dem Übersichtsartikel von G. N. Jackson (RF Sputtering, Thin Solid Films, Vol. 5, 1970, S. 209-246) besprochen. Beispielhaft zu erwähnen sind auch die Methoden des sogenannten "spin coatings" (Rotationsbeschichtung), wie es in dem Aufsatz von N. Sahu et al. (Fundamental understanding and modeling of spin coating process: A review, Indian Journal of Physics, Vol. 83 (4), 2009, S.493-502) beschrieben ist und der Atomlagenabscheidung (engl.: atomic layer deposition, ALD) wie es in der DE 25 53 048 A1 vorgestellt ist. Die hier erwähnten, sowie alle weiteren möglichen Techniken zur Abscheidung fester dünner Filme (thin solid films) sind dem Fachmann bekannt. Der erfinderische Multischichtkatalysator ist nicht an eine bestimmte Art der Herstellung dünner Schichten gebunden.

### Laserablation

Das Lasermuster wurde mit einem ROFIN-BAASEL Lasertech-System durchgeführt, das mit Diode Pumped Solid State (DPSS) Nd:YVO₄-Lasern ausgestattetwar . Das System verfügte sowohl über Nanosekunden als auch Pikosekunden Laserquellen mit Wellenlängen im sichtbaren (VIS) und infraroten (IR) Spektrum. Ein integriertes Kamerasystem gewährleistete eine präzise optische Qualitätskontrolle und eine exakte Positionierung des Laserstrahls während des Strukturierungsprozesses.

Die für die Experimente verwendeten Substrate 2 hatten eine maximale Größe von 300 mm x 300 mm und umfassten sowohl Glas- als auch Dünnschichtsubstrate. Das System ermöglichte eine Bearbeitungsgeschwindigkeit von bis zu 1200 mm/s bei einer Positioniergenauigkeit von weniger als 5 µm, was die Erstellung hochpräziser Stapelgeometrien des Mehrschichtkatalysators 1 erlaubte. Die Laserstrahl-Spotgröße war innerhalb eines Bereichs von 20 µm bis 80 µm im Durchmesser einstellbar, um spezifische Anforderungen zu erfüllen.

Das System verfügt über einen Nanosekunden- sowie Pikosekunden Laser mit Pulswiederholraten zwischen 20 kHz und 400 kHz beim Nanosekundenlaser und zwischen 50 kHz und 200 kHz beim Pikosekundenlaser. Die Nanosekundenlaser erreicht eine maximale Pulsenergie von 41 µJ und der Pikosekundenlaser eine maximale Pulsenergie von 60 µJ mit Puls-Spitzenleistungen im Bereich von 2,7 kW bis 8000 kW. Die Strahltaille, Rayleigh-Länge und Fokus-Tiefe wurden angepasst, um eine präzise Energieübertragung auf das Substrat zu gewährleisten. Diese Parameter wurden optimiert, um eine qualitativ hochwertige Laser-Material-Wechselwirkung zu erreichen, die für die untersuchten katalytischen Anwendungen entscheidend war.

Das System war mit einer Vielzahl von Materialien kompatibel, die als Dünnschichten abgeschieden werden konnten, sofern deren Schmelzpunkte für die Laserbearbeitungsbedingungen geeignet waren. Die Prozessparameter wurden für jedes Material optimiert, um eine effektive Strukturierung und eine hohe Reproduzierbarkeit über alle Proben hinweg sicherzustellen.

Detaillierte instrumentelle Parameter für Wellenlänge, Pulsbreite und Fokus-Tiefe sind in Tabelle 1 dargestellt. Anpassungen wurden vorgenommen, um gewünschte Laser-Material-Wechselwirkungen zu erreichen, die speziell auf die katalytischen Anwendungen abgestimmt waren.

### Katalytische Aktivitätsmessung

Die katalytische Leistung des Mehrschichtkatalysators 1 wurde mit einem kommerziellen Berty Reaktor bewertet. Der Reaktor ist für einen Betrieb bei Drücken von bis zu 50 bar und Temperaturen von bis zu 525°C ausgelegt.

Die Zusammensetzung der Reaktionsprodukte wurde mittels Gaschromatographie analysiert. Der Gasstrom am Reaktorausgang wurde entnommen und durch ein Gaschromatographen geleitet. CO₂-Umwandlung und Produkte wurden quantifiziert.

### Rastertransmissionselektronenmikroskopie und Elementaranalyse

Die physikochemischen Eigenschaften der Schnittstelle des Cu/ZnO-Mehrschichtkatalysators 1 wurden mittels Rasterelektronenmikroskopie (REM) untersucht.

## Patentansprüche

1. Mehrschichtkatalysator (1), aufweisend:
- ein Substrat (2) sowie
- einen auf dem Substrat (2) angeordneten Stapel (4) mit mindestens 10 übereinander angeordneten Schichten, wobei benachbarte Schichten (4) unterschiedliche Materialien aufweisen und wobei der Stapel (4) eine Vielzahl von Aussparungen (5) aufweist oder
eine Vielzahl von Stapeln (4', 4", 4‴, 4ʺʺ) auf dem Substrat (2) angeordnet ist und
wobei der Mehrschichtkatalysator (1) ein Stapeloberfläche-zu-Stapelvolumen-Verhältnis aus einer freiliegenden Oberfläche des Stapels (4) oder der Stapel (4', 4", 4‴, 4ʺʺ) zu einem Volumen des Stapels (4) oder der Stapel (4', 4", 4‴, 4ʺʺ) aufweist, das zwischen
100 1/m und 400.000.000 1/m, insbesondere zwischen 10.000 1/m und 4.000.000 1/m beträgt.

2. Mehrschichtkatalysator (1) nach Anspruch 1, wobei jede der Schichten eine Schichtdicke im Bereich von einer Atomlage bis 50 nm aufweist, insbesondere wobei jede der Schichten eine Schichtdicke im Bereich von 0,5 nm bis 30 nm aufweist.

3. Mehrschichtkatalysator (1) nach einem der vorhergehenden Ansprüche, wobei die Materialien der Schichten aus Metallen; Metalloxiden; Metall- oder Kohlenstoffnitriden; Metallcarbiden; Legierungen; insbesondere aus Metallen und Metalloxiden ausgewählt sind.

4. Mehrschichtkatalysator (1) nach einem der vorhergehenden Ansprüche, wobei die Materialien der Schichten aus Metalloxid und Metall ausgewählt sind.

5. Mehrschichtkatalysator (1) nach einem der vorhergehenden Ansprüche, wobei die Schichten Metallschichten und Metalloxidschichten aufweisen, wobei im Stapel (4) oder in den Stapeln (4', 4", 4‴, 4ʺʺ) jeweils eine Metallschicht und eine Metalloxidschicht abwechselnd übereinander angeordnet sind.

6. Mehrschichtkatalysator (1) nach Anspruch 5, wobei die Metallschicht Kupfer aufweist und wobei die Metalloxidschicht Zinkoxid aufweist.

7. Mehrschichtkatalysator (1) nach einem der vorhergehenden Ansprüche, wobei die Vielzahl von Stapeln (4', 4", 4‴, 4ʺʺ) auf dem Substrat (2) eine Stapeldichte zwischen 10.000/cm² und 10.000.000.000/cm² aufweisen.

8. Mehrschichtkatalysator (1) nach einem der vorhergehenden Ansprüche, wobei die Vielzahl von Aussparungen (5) des wenigstens einen Stapels (4) auf dem Substrat (2) eine Aussparungsdichte zwischen 10.000/cm² und 10.000.000.000/cm² aufweist.

9. Mehrschichtkatalysator (1) nach Anspruch 8, wobei mindestens eine Aussparung (5), insbesondere jede der Vielzahl von Aussparungen (5) ein Durchgangsloch durch den Stapel (4) bildet.

10. Mehrschichtkatalysator (1) nach einem der Ansprüche87 oder 9, wobei mindestens eine Aussparung (5) ein Sackloch in dem Stapel (4) bildet.

11. Mehrschichtkatalysator (1) nach einem der vorhergehenden Ansprüche, aufweisend eine Vielzahl von auf dem Substrat (2) angeordneten Stapeln (4', 4", 4‴, 4ʺʺ) mit jeweils einer Vielzahl von Aussparungen (5).

12. Mehrschichtkatalysator (1) nach einem der vorhergehenden Ansprüche, wobei jeder Stapel (4, 4', 4", 4"', 4ʺʺ wenigstens ein Paar einander gegenüberliegender freiliegender Oberflächenabschnitte aufweist und wobei ein maximaler Abstand zwischen dem Paar der gegenüberliegenden freiliegenden Oberflächenabschnitten im Bereich von 1 nm bis 50 µm liegt, insbesondere wobei der Abstand zwischen den zwei gegenüberliegenden freiliegenden Oberflächenabschnitten im Bereich von 10 nm bis 50 µm liegt, speziell wobei der Abstand zwischen den zwei offenen Oberflächenabschnitten im Bereich von 1 µm bis 50 µm liegt.

13. Mehrschichtkatalysator nach einem der vorhergehenden Ansprüche, wobei eine Höhe des Stapels (4) oder der Stapel (4', 4", 4‴, 4"") zwischen 0,5 µm und 30 µm beträgt.

14. Verfahren zur Umwandlung von CO₂ unter Verwendung von Wasserstoff in Gegenwart eines Mehrschichtkatalysators (1) gemäß Anspruch 1 bis 13.
